(19) Europäisches Patentamt
European Patent Office
Office européen des brevets

(11) **EP 3 594 320 A1**

(12) **EUROPEAN PATENT APPLICATION**

(43) Date of publication:
**15.01.2020 Bulletin 2020/03**

(51) Int Cl.:
***C12M 1/00*** *(2006.01)*

(21) Application number: **18182735.3**

(22) Date of filing: **10.07.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Université de Liège
4000 Liège (BE)**

(72) Inventors:
• **BLANCHARD, Gersande
4557 Ramelot (BE)**
• **DELILLE, Bruno
4000 Liège (BE)**
• **DARCHAMBEAU, François
4560 Ocquier (BE)**

(74) Representative: **Savoye, Anne
ICOSA Europe
Rue d'Oultremont 37
1040 Bruxelles (BE)**

(54) **APPARATUS AND METHOD FOR THE PRODUCTION OF MICROALGAE**

(57)     This apparatus (30) for the production of microalgae comprises a vessel (1) intended to receive a culture medium of the microalgae, and a lighting device (6) intended to be positioned in the vessel inside the culture medium. The lighting device (6) is configured to emit light at least in a range of wavelengths useful for the photosynthesis of the microalgae. The apparatus comprises a control system (15) for automatically controlling the light intensity emitted by the lighting device (6) as a function of the concentration of microalgae in the vessel (1).

Fig.1

EP 3 594 320 A1

## Description

The present invention relates to a device and a method for producing microalgae, such as Spirulina (*Arthrospira platensis*). In particular, the invention relates to a device and a method for autonomous and domestic production of fresh microalgae.

The use of microalgae to sustain food transition is of great interest, in particular with regard to the process of increasing substitution of animal proteins. Worldwide, about 150 different species of microalgae are cultivated. *Arthrospira platensis,* better known as Spirulina, is the most widely cultivated microalgae, representing 50% of world production. Spirulina is a super-food with highly ecological and nutritional quality that can target a large part of the population. This filamentous blue-green algae, or cyanobacterium, is naturally found in tropical lakes and is highly nutritious. The biomass is comprised of more than 60% protein, and the protein is nutritionally complete containing all the essential amino acids. It also has minerals including iron and selenium in significant amounts, vitamins A, D, E, B1, B2, B3, B6, B7, B8, B12 and K, natural antioxidants including phycocyanin, essential fatty acids, sterols, etc. Having no cell wall, the organism is easily digested.

The majority of commercially available microalgae, in particular Spirulina, is produced in plant-scale cultivation, in open systems and utilizing natural conditions, such as sunlight, ambient temperature and timely seasons. After harvesting, the microalgae must be transformed to ensure long-time conservation of the commercial end-product. Each step of the transformation process, including rising, pressing, extrusion, drying, usually decreases the nutritional quality of the microalgae. In addition, for imported microalgae, there may be concerns relating to traceability or contamination. For these reasons, there is a growing demand for local production and, more recently, home production, of fresh microalgae allowing daily and high-quality consumption.

For the production of microalgae, it is also known to use photobioreactors, which are closed systems allowing photosynthetic microorganisms to develop in the presence of light energy. Such closed systems, in comparison to open ones, have many advantages among which higher productivity, lower water consumption and lower risk of contamination. However, with known photobioreactors, it is not possible to optimize productivity for home use purposes, in particular when the culture concentration fluctuates as is the case in photobioreactors allowing daily harvesting of microalgae.

It is these drawbacks that the invention is intended more particularly to remedy by proposing a device and a method for the production of microalgae, making it possible to cultivate and consume daily fresh and safe microalgae, with an optimized productivity rate at each step of the culture.

For this purpose, a subject of the invention is an apparatus for the production of microalgae, comprising a vessel intended to receive a culture medium of the microalgae and a lighting device intended to be positioned in the vessel inside the culture medium, the lighting device being configured to emit light at least in a range of wavelengths useful for the photosynthesis of the microalgae, characterized in that the apparatus comprises a control system for automatically controlling the light intensity emitted by the lighting device as a function of the concentration of microalgae in the vessel.

Thanks to the invention, the light intensity emitted by the lighting device and received by the microalgae is adjusted in real-time according to the concentration of microalgae in the vessel. In this way, it is possible to optimize the productivity rate at each step of the culture. In particular, at the beginning of the culture, when the concentration of microalgae is low, the light intensity emitted by the lighting device is controlled to avoid photolysis of cells. Then, during the growing phase of the microalgae, the light intensity emitted by the lighting device is continuously adapted to correspond to the photosynthetic needs of the microalgae. During the harvesting phase of the microalgae, after each extraction of a volume of microalgae, the light intensity emitted by the lighting device is controlled to be adjusted to the new lower concentration of microalgae remaining in the vessel.

According to one aspect of the invention, the control system comprises at least one light sensor intended to receive a light intensity depending on the concentration of microalgae in the vessel, and a processing unit configured to adjust the light intensity received by the light sensor at a predefined value of light intensity by controlling the light intensity emitted by the lighting device. In this way, the control system controls the light intensity emitted by the lighting device as a function of the light intensity received by the light sensor, according to a predefined relationship. In particular, the relationship is derived from the Beer-Lambert law, and adapted and optimized for the device.

The light sensor is advantageously arranged to receive light emitted by the lighting device and that has passed through the culture medium. With such an arrangement, the light intensity received by the light sensor is correlated to the concentration of microalgae in the vessel.

According to one embodiment, the processing unit is configured to determine the concentration of microalgae in the vessel by comparison between the light intensity emitted by the lighting device and the light intensity received by the light sensor. According to a particular embodiment, the processing unit is configured to determine once a day, at night, the concentration of microalgae in the vessel by comparison between the light intensity emitted by the lighting device at its maximum intensity and the light intensity received by the light sensor. Based on this comparison and the position of the light sensor relative to the lighting device, the absorbance of the culture medium can be determined. The concentration of microalgae in the vessel can then be computed from the absorbance according to the Beer-Lambert law. It is noted that the light intensity received by the light sensor must be corrected for the ambient light. Then, the ambient light intensity is measured by turning off the lighting device during a few seconds. This is done automatically by the processing unit. The processing unit computes the light intensity received from the lighting device by subtracting the ambient light intensity from the total light intensity.

According to one embodiment, the lighting device is positioned centrally inside the vessel. The lighting device is advantageously configured to emit light in all radial directions from the center of the vessel. In this way, the light emitted by the lighting device can be distributed homogeneously in the culture medium received in the vessel and can reach optimally the microalgae for their photosynthesis.

According to one embodiment, the light sensor is positioned on a peripheral wall of the vessel. When the peripheral wall of the vessel is transparent, the light sensor is positioned preferably on an outer face of the peripheral wall of the vessel, so as to avoid microalgae accumulation on the light sensor and calibration problems. The light sensor can be a phototransistor, sensitive to visible light.

Preferably, the position of the light sensor with respect to the lighting device is selected in such a manner that the light intensity received by the light sensor is representative of a mean concentration of the microalgae in the vessel. For example, in the case of a cylindrical vessel, when the lighting device is positioned at the center of the cylindrical vessel, extending along the longitudinal axis thereof, and the light sensor is positioned on the peripheral wall of the cylindrical vessel, the light intensity received by the light sensor is representative of a mean concentration of the microalgae in the vessel, provided that the concentration of the microalgae in the vessel is homogeneous, which can be ensured by appropriately mixing the culture medium received in the vessel.

According to one embodiment, the light sensor is configured to receive light not only from the lighting device of the apparatus, but also from external light sources emitting light in a range of wavelengths useful for the photosynthesis of the microalgae, including in particular natural sunlight during the day. Then, the control system comprising the light sensor and the processing unit can control the light intensity emitted by the lighting device in an optimized manner, in such a way that the light intensity emitted by the lighting device complements the light intensity emitted by the surrounding external light sources to correspond to the photosynthetic needs of the microalgae.

According to an advantageous feature, in order to maximize the productivity, the lighting device is configured to emit light with wavelengths matching the pigment specificities of the microalgae. The light sensor is selected to be sensitive to at least these wavelengths. In one embodiment, the lighting device may be configured to emit a white light, so that all the pigments of the microalgae are involved in photosynthesis. As a variant, the lighting device may be configured to emit a colored light, and the light color may then be dictated by both the pigment specificities of the microalgae and the desired appearance of the apparatus which must be appetizing for home use. For the culture of Spirulina (*Arthrospira platensis*), the lighting device is advantageously configured to emit a white light comprising wavelengths in the range 400 nm-700 nm.

According to one embodiment, the lighting device comprises at least one LED (Light-Emitting Diode) as a light source. The use of LEDs as light sources is advantageous, in particular due to their high lifetime and affordability. In one embodiment, the lighting device may comprise a plurality of LEDs distributed along a transparent cylindrical tube positioned at the center of the vessel. Preferably, each light source of the lighting device has an angle diffusion of at least 120°. According to one embodiment, the lighting device comprises at least one LED emitting white light with a CCT (Color Correlated Temperature) of between about 3000K and 5000K. The use of LEDs with such a CCT is particularly well suited for the culture of Spirulina (*Arthrospira platensis*).

According to one embodiment, the lighting device is configured to be activated according to a daily ON/OFF cycle, and during the ON period the light intensity emitted by the lighting device is regulated according to the concentration of microalgae in the vessel. Advantageously, the OFF period is during the night. According to one embodiment, the processing unit is configured to control both the ON/OFF switching of the lighting device and the light intensity emitted by the lighting device during the ON period as a function of the concentration of microalgae in the vessel.

According to one aspect of the invention, the apparatus comprises a harvesting device configured to ensure filtration of microalgae from a volume of the culture medium and allow the remaining culture medium to return in the vessel. In this way, the harvesting device ensures filtration of microalgae from a given volume of the culture medium taken in the vessel, while allowing nutrients and water to return to the culture medium left in the vessel.

According to one embodiment, the harvesting device comprises a membrane pump configured to allow intake of a volume of the culture medium from the vessel, and a cup configured to receive the microalgae to be filtered, transferred from the membrane pump. In one embodiment, the membrane pump and the cup of the harvesting device are housed in a cover intended to be positioned on the vessel. Preferably, the cup is removably housed in the cover, so that the filtered microalgae can be collected easily.

According to one embodiment, the output of the membrane pump of the harvesting device is connected to a filtration membrane having a first pore size, notably of the order of 300-400 μm, which is located above the cup, whereas the cup has a second pore size less than the first pore size, notably of the order of 30-60 μm. In an advantageous manner, the membrane pump and the filtration membrane allow intake of the culture medium from the vessel and its transfer in the cup without damaging cell morphology, ensuring that the nutritive quality of the microalgae is preserved.

According to one embodiment, the apparatus comprises a processing unit configured to activate the harvesting device and allow automatic filtering of a given volume of the culture when a corresponding instruction is applied by a user. In particular, the apparatus may be provided with a harvesting button on which a user can click, where each click on the harvesting button triggers automatic filtering of a given volume of the culture medium. Advantageously, the filtered volume may be adjusted by clicking several times on the harvesting button.

According to one feature of the invention, the apparatus comprises a heating device intended to adjust the temperature of the culture medium received in the vessel. According to another feature of the invention, the apparatus comprises an aeration and mixing device intended to ensure homogeneity of temperature and homogeneity of microalgae distribution in the culture medium received in the vessel, and to provide atmospheric $CO_2$ and remove excess dissolved $O_2$ in/from the culture medium.

According to one embodiment, the apparatus comprises a processing unit configured to control all of the lighting device, the harvesting device, the heating device, the aeration and mixing device.

According to an advantageous feature, electronic elements of the apparatus are housed in a removable part intended to be fitted to the vessel. In this way, the vessel can be easily washed, once separated from the removable part, without risk of impairing the electronic elements. Electronic elements of the apparatus include at least the processing unit and an electric transformer intended to supply power to the electric components of the apparatus, such as the lighting device, the harvesting device, the heating device, the aeration and mixing device. The membrane pump of the harvesting device and the aeration pump of the aeration and mixing device may also be housed in the removable part. In particular, the removable part may be a removable cover intended to be positioned on the top of the vessel so as to close it.

The invention also relates to a method for the production of microalgae by means of an apparatus as described above.

Another subject of the invention is a method for the production of microalgae, comprising:

- filling a vessel with a culture medium of the microalgae;
- activating a lighting device positioned in the vessel inside the culture medium, the lighting device being configured to emit light at least in a range of wavelengths useful for the photosynthesis of the microalgae;
- automatically controlling the light intensity emitted by the lighting device as a function of the concentration of microalgae in the vessel.

According to one embodiment, the automatic control of the light intensity emitted by the lighting device as a function of the concentration of microalgae in the vessel is performed by controlling the light intensity emitted by the lighting device so as to adjust the light intensity received by at least one light sensor at a predefined value of light intensity depending on the concentration of microalgae in the vessel.

According to one feature of the invention, the light sensor is arranged to receive light emitted by the lighting device and that has passed through the culture medium. Then, the light intensity received by the light sensor depends on the light absorbance of the culture medium, which is related to the concentration of microalgae in the vessel. The assessment of the light absorbance of the culture medium is corrected to take into account the ambient light intensity.

In an advantageous embodiment, the automatic control of the light intensity emitted by the lighting device as a function of the concentration of microalgae in the vessel is performed during a growing phase of the microalgae and/or during a harvesting phase of the microalgae. It is thus possible to avoid photolysis of cells under low concentration of microalgae in the growing phase and/or to adapt to each change in the concentration of microalgae resulting from an extraction of microalgae during the harvesting phase. The automatic control of the light intensity emitted by the lighting device also takes into account the ambient light intensity.

Features and advantages of the invention will become apparent from the following description of an embodiment of an apparatus and a method for the production of microalgae according to the invention, this description being given merely by way of example and with reference to the appended drawings in which:

Figure 1 is a perspective view of an apparatus for the production of microalgae according to the invention;

Figure 2 is a cross section along the plane II of Figure 1;

Figure 3 is a cross section along the line III-III of Figure 2; and

Figure 4 is a graph showing the evolutions, as a function of time during the growing phase, both of the light intensity emitted by the lighting device of the apparatus of Figures 1 to 3 and of the concentration of microalgae in the vessel of the apparatus, in an illustrative example of a production cycle of Spirulina (*Arthrospira platensis*) by means of the apparatus.

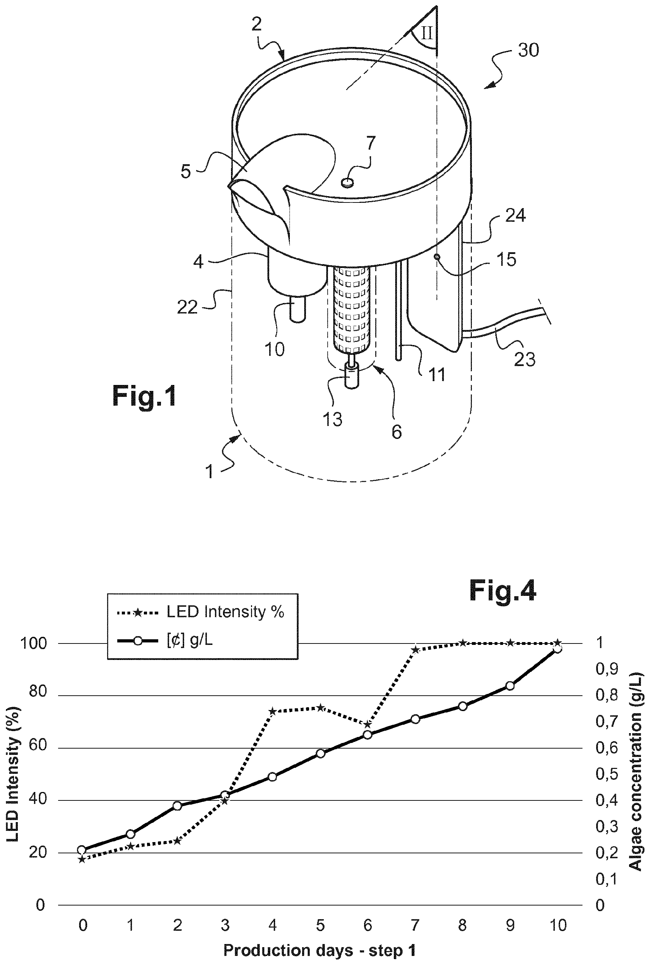

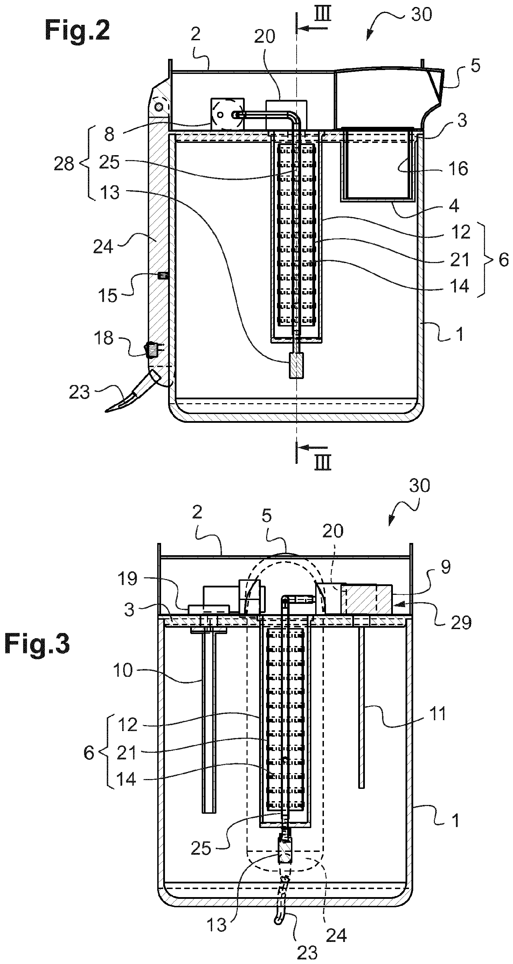

As shown in Figures 1 to 3, the apparatus 30 according to the illustrative embodiment of the invention comprises a cylindrical vessel 1, intended to contain an alkaline culture medium of the microalgae, and a cover 2 located on the upper part of the vessel 1. The cover 2 is intended to house and protect technical components needed for the culture of the microalgae. The cover 2 is designed to close the vessel 1 so as to lower evaporation and protect the culture against contamination. The apparatus 30 also has a general power supply system 23 (of 230V) and an electric transformer 20 housed in the cover, which is intended to power supply several components of the apparatus at a lower voltage (24V or 12V). The apparatus 30 is provided with a main switch button 18 for switching the apparatus ON/OFF.

In an advantageous manner, the walls of the vessel 1 are transparent so that photosynthesis of the microalgae can occur with the light from both internal light sources positioned in the vessel and external light sources. As a variant, the walls of the vessel 1 may not be transparent, in which case the apparatus 30 involves photosynthesis of the microalgae with the light of internal light sources only. In this embodiment, the vessel 1 is made of glass, which has the advantages of being an inert material, resistant to culture medium characteristics, offering a weakly adhering surface to microalgae accumulation, easy to wash and sustainable. Of course, transparent materials other than glass may also be considered for the vessel 1.

The apparatus 30 comprises a lighting device 6 which is fixed on a lower part 3 of the cover 2 in such a way that, when the cover 2 is fitted to the vessel 1, the lighting device 6 is immersed in the culture medium received in the vessel 1. In this embodiment, the lighting device 6 comprises an outer transparent cylindrical tube 12 hermetically closed at the bottom, and an inner transparent cylindrical tube 21 placed inside the outer tube 12. A ribbon of LEDs 14 is fixed onto the inner tube 21 so that the LEDs 14 are distributed along the tubes 12, 21, in the volume defined between the two tubes. In this example, the cylindrical tubes 12 and 21 are advantageously made of PMMA (poly(methyl methacrylate)) compatible with alimentary purpose.

In this illustrative embodiment, the lighting device 6 is positioned at the center of the vessel 1 when the cover 2 is fitted to the vessel 1. In this way, the light emitted by the lighting device 6 is distributed homogeneously in the culture medium received in the vessel 1 and can reach the microalgae optimally for their photosynthesis. The wavelengths of the LEDs 14 are adapted to the pigment specificities of the microalgae to be produced in the apparatus. For example, in the case where the apparatus 30 is used for the production of Spirulina (*Arthrospira platensis*), the LEDs 14 are advantageously white LEDs with a CCT of 4100 K, so that all Spirulina pigments are involved in photosynthesis.

The apparatus 30 further comprises a control system for automatically controlling the light intensity emitted by the lighting device 6 as a function of the concentration of microalgae in the vessel 1. As visible in Figures 1 to 3, the control system comprises a light sensor 15, positioned on the outer face 22A of the peripheral wall 22 of the vessel 1, and a processing unit 19, housed in the cover 2. For example, the light sensor 15 may be a silicon NPN epitaxial planar phototransistor, sensitive to visible light much like the human eye, having a peak sensitivity at 570 nm and an angle of half sensitivity of 20°.

In accordance with the invention, the processing unit 19 is configured to control the light intensity emitted by the lighting device 6 so as to adjust the light intensity received by the light sensor 15 at a predefined value of light intensity depending on the concentration of microalgae in the vessel 1. In this example, the processing unit 19 is further configured to activate the lighting device 6 according to a daily ON/OFF cycle. According to a specific example, the characteristics of the lighting device 6 are the following: 60 LEDs per meter; 14,4 W/m; and the control parameters of the lighting device 6 are: ON/OFF 16 hours/8 hours between 6 am and 10 pm and light intensity adapted according to the concentration of microalgae in the vessel 1 during the ON period.

As visible in Figure 1, the light sensor 15 is attached to a vertical gutter 24 extending along the height of the peripheral wall 22 of the vessel 1. More precisely, the light sensor 15 is placed under the gutter 24 and against the peripheral wall 22 of the vessel 1. In this way, the light sensor 15 can capture total light corresponding to both light emitted by the lighting device 6 and that has passed through the culture medium received in the vessel 1, and ambient light emitted by external light sources also useful for the photosynthesis of the microalgae, such as natural sunlight during the day. In order to distinguish the light emitted by the lighting device 6 from the ambient light emitted by external light sources, the processing unit 19 turns off the lighting device 6 for a few seconds. At that time, the light sensor 15 receives only the ambient light emitted by external light sources. By subtracting the ambient light emitted by external light sources from the total light, the processing unit 19 computes the light emitted by the lighting device 6 and that has passed through the culture medium.

This configuration allows an optimal light utilization taking into account surrounding external light sources. In an advantageous manner, with such a configuration, the processing unit 19 can control the light intensity emitted by the lighting device 6 in such a way that the light intensity emitted by the lighting device 6 complements the light intensity emitted by the surrounding light sources to result in a light intensity received by the light sensor 15 adjusted to the predefined value of light intensity.

Depending on the light intensity received by the light sensor 15, the processing unit 19 pilots the light intensity of the LEDs 14 of the lighting device 6 according to a predefined equation, where the relationship between LED intensity and microalgal concentration in the vessel corresponds to the photosynthetic needs of the microalgae at a given temperature of the culture medium. Thus, the processing unit 19 pilots the light intensity of the LEDs 14 so that the light intensity received by the microalgae at each time is adjusted according to the photosynthetic needs of the microalgae at this time and at a given temperature of the culture medium. An exponential relationship between LED intensity and microalgal concentration is particularly suitable.

In particular, according to a particular embodiment, the light intensity received from the lighting device 6 ($IR_{cor}$) is calculated by subtracting from the total light intensity received by the light sensor 15 (IR) the ambient light intensity ($I_{amb}$) measured by the light sensor 15 when the lighting device 6 is turned off. This is expressed by equation (1):

$$IR_{cor} = IR - I_{amb} \quad (1)$$

The light absorbance (A) of the culture medium is calculated from the light intensity emitted by the lighting device ($I_{LED}$) and the light intensity received from the lighting device ($IR_{cor}$) according to equation (2):

$$A = \log \frac{I_{LED}}{IR_{cor}} \quad (2)$$

The concentration of microalgae (c) is derived from the distance (l) between the lighting device 6 and the light sensor 15, and the absorptivity ($\varepsilon$) of the microalgae solution according to equation (3):

$$c = \frac{A}{\varepsilon . l} \quad (3)$$

As a variant, the relationship between the absorbance and the microalgal concentration may be obtained by a linear regression after calibration with samples having different concentrations.

The processing unit 19 is also configured to automatically regulate the temperature of the culture medium received in the vessel 1, by controlling a heating device 10 also fixed on the lower part of the cover 2 and consequently immersed in the culture medium when the cover 2 is fitted to the vessel 1. The heating device 10 is in the form of a heating resistance, preferably made of stainless steel in accordance with alimentary legislation. The heating device 10 has overheated self-controlled security. The processing unit 19 is configured to control the heating device 10 so as keep the optimum temperature of culture without cells damaging, in a cost-effective way. According to a specific example, the control parameters of the heating device 10 are: heating power adapted to keep the culture medium at 35°C +/- 1°C.

The processing unit 19 is also configured to control an aeration and mixing device 28 intended to ensure homogeneity of temperature, homogeneity of microalgae distribution and gas exchange in the culture medium received in the vessel 1. The aeration and mixing device 28 comprises an air pump 8 having an entrance antibacterial air filter, which is housed in the cover and connected to an aeration element 13 by means of a flexible tube 25. The flexible tube 25 passes through the internal space of the inner tube 21 of the lighting device 6 and is hermetically connected to the aeration element 13. The aeration element 13 extends beyond the ends of the tubes 12, 21 of the lighting device 6 so as to be immersed inside the culture medium when the cover 2 is fitted to the vessel 1. The aeration element 13 is preferably in stainless steel in accordance with alimentary legislation. The processing unit 19 is configured to control the aeration and mixing device 28 so as keep cells in suspension in the culture medium, sustain gas exchanges and in particular $CO_2$ supply to the photosynthetic organisms and removal of excess dissolved $O_2$, and ensure temperature homogeneity in the culture medium. Supply of $CO_2$ also maintains the pH of the culture medium above 10. This pH level favors growth of Spirulina (*Arthrospira platensis*) and prevents growth of other microbial communities. Removal of excess oxygen prevents buildup of hyperoxic conditions.

The apparatus 30 also has a harvesting device 29, housed inside of the cover 2, configured to ensure filtration of microalgae from a given volume of the culture medium taken in the vessel 1, while allowing nutrients and water to return to the culture medium left in the vessel. The harvesting device 29 comprises a self-priming membrane pump 9 connected to an intake culture tube 11 so as to allow intake of a volume of the culture medium from the vessel 1. The harvesting device 29 also comprises a cup 16 having stainless steel bottom and cylindrical wall, which is housed inside the cover 2 and protected by a hood 5 with hinge. The cup 16 is removably held on a filter support 4 defined by the cover 2, so that the filtered microalgae can be collected easily by separating the cup 16 from the cover 2.

The configuration of the harvesting device 29 is such that, after a volume of the culture medium has been pumped from the vessel 1, the microalgae are first filtered passing through a filtering membrane having a first pore size of the order of 315 $\mu$m, which is connected to the membrane pump 9 and located above the cup 16. This step allows the culture sludge to be rejected from the microalgae. Then, the culture is transferred and filtered through the cup 16 whose bottom and wall have a second pore size less than that of the filtering membrane, notably of the order of 55 $\mu$m or 30 $\mu$m. In this way, the harvesting device 29 allows intake of the culture medium from the vessel 1 and its transfer in the cup 16 without damaging cell morphology, thus preserving the nutritive quality of the microalgae.

Here again, the processing unit 19 is configured to control the harvesting device 29. More specifically, the apparatus is provided with a harvesting button 7 on which a user can click, so that for each click the processing unit 19 triggers filtering of a given volume of the culture medium, for example 1L of the culture medium. The filtered volume can be adjusted by clicking several times on the harvesting button 7, for example each click corresponding to 1L. Optionally, the apparatus 30 may comprise a harvest indicator (not shown in the figures) to indicate the right harvesting time.

As explained above, important parameters of the culture are automatically controlled by the processing unit 19, including: homogeneity of temperature; temperature of the culture medium received in the vessel 1; ON/OFF switching of the lighting device 6 and light intensity emitted by the lighting device 6 during the ON period as a function of the concentration of microalgae in the vessel 1; volume of the culture medium extracted by means of the harvesting device 29. In particular, since the processing unit 19 controls both the lighting device 6 and the harvesting device 29, light control can be performed at any time of the culture, allowing a wide freedom of use in terms of moment and quantities of microalgae to be harvested.

By way of example, in the case of the production of Spirulina (*Arthrospira platensis*) by means of the apparatus 30, a full cycle of production takes place during about 40 days long and is divided in two distinct phases, namely a growing phase and a harvesting phase. The growing phase duration is comprised between 7 and 10 days. The harvesting phase is about 30 days, which corresponds to a recommended cure time of Spirulina. As illustrated with the apparatus 30, the invention has the advantage that it ensures an optimized productivity rate at each step of the culture, both during the growing phase and during the harvesting phase. The processing unit 19 has all information, obtained from the heating device 10, the light sensor 15, the aeration and mixing device 28, the harvesting device 29, in order to adjust in real-time the light intensity emitted by the lighting device 6 and received by the microalgae according to the concentration of microalgae in the vessel 1 and the temperature of the culture medium.

In particular, at the beginning of the culture, when the concentration of microalgae is low, the light intensity emitted by the lighting device 6 is controlled to avoid photolysis of cells. Then, during the growing phase, the light intensity emitted by the lighting device 6 is continuously adapted to correspond to the photosynthetic needs of the microalgae. During the harvesting phase, after each extraction of a volume of microalgae by means of the harvesting device 29, the light intensity emitted by the lighting device 6 is controlled to be adjusted to the new lower concentration of microalgae remaining in the vessel 1, which can be computed by the processing unit 19.

By way of example, Figure 4 shows the evolutions, as a function of time during the growing phase, of the light intensity emitted by the lighting device 6 and of the concentration of microalgae in the vessel 1, in an illustrative example of production of Spirulina by means of the apparatus 30. In this example, the light intensity emitted by the lighting device 6 is automatically regulated by the processing unit 19 so as to meet the photosynthetic needs of Spirulina at each time.

An illustrative example of a method for the production of Spirulina (*Arthrospira platensis*), by means of the apparatus 30, comprises steps as described below.

First, to initiate the growing phase, the vessel 1 of the apparatus 30 is filled with 9L of demineralized water. Then, the content of a first nutrient bag is added into the water contained in the vessel 1. For example, the components of the first nutrient bag are the following: $NaHCO_3$, $NaCl$, $KNO_3$, $K_2SO_4$, $MgSO_4$ $7H_2O$, $NH_4H_2PO_4$, $CaCl_2$, $CO(NH_2)_2$. In addition, $FeSO_4$ is provided to feed the microalgae.

In order to activate the heating device 10 and the aeration and mixing device 28, the apparatus 30 is then switched on, by means of the main switch 18. After dissolution, an inoculation strain (1L at 2g/L concentration) is gently added into the culture medium received in the vessel 1. The Spirulina growing phase is started with 10L of culture at 0,2g/L concentration. The inoculation strain may be provided in a bottle of 1L at a concentration of 2g/L microalgae. It may also be provided in another form, for example concentrated under fresh Spirulina biomass in a protected atmosphere, allowing extended lifetime of the inoculation strain. During the growing phase, the microalgae concentration evolves from clear green to dark green in 7 to 10 days.

At the end of the growing phase is the beginning of the harvesting phase, corresponding to 30 days of fresh Spirulina consumption. Each day, a maximum of 30% in volume of the culture medium is harvested by clicking on the harvesting button 7, according to the need of a user. Each click allows to automatically filter 1L of the culture medium. The remaining culture medium is recycled and returned into the vessel 1. The filtered microalgae is available in the removable cup 16 located in the cover 2 of the apparatus 30, by opening the hinged hood 5. After a manual step allowing to press the filtered microalgae, fresh Spirulina is directly consumed or added into culinary preparations. Fresh Spirulina can be preserved during 48 hours at 4-5°C in a refrigerator or longer preserved by freezing or drying.

To compensate the loss of nutrients in the culture medium and sustain productivity, nutrients are regularly added to the culture medium during the harvesting phase. For example, the components of a second nutrient bag needed for the 30 days of the harvesting phase are the following: $NaHCO_3$, $NaNO_3$, $NH_4H_2PO_4$, $K_2SO_4$, $MgSO_4$ $7H_2O$. $FeSO_4$ is provided to feed or enrich the culture medium. Nutrient bags are designed to protect nutrients against oxidation and humidity. Evaporation is also compensated by adding demineralized water. After 30 days of harvesting phase, new inoculation strain and culture medium may be provided to start a new cycle.

In an advantageous manner, nutrient bag with higher concentration of one or several specific compounds (e.g. iron, zinc, selenium) may be provided in order to respond to specific nutritional deficiencies of a user. These bags support enrichment of the culture in a specific compound, resulting in higher concentration of a specific compound in fresh Spirulina.

In accordance with the invention, during both the growing phase and the harvesting phase of the production cycle, in order to avoid photo-inhibition phenomenon, the light intensity emitted by the lighting device 6 is automatically controlled by the processing unit 19 according to the microalgae concentration in the vessel 1. The light sensor 15 is calibrated to receive a predefined value of light intensity in accordance with specific needs of Spirulina. As illustrated in the graph of Figure 4, at low microalgae concentration, the light intensity needed to achieve the predefined value of light intensity to be received by the light sensor 15 is low. Indeed, at low microalgae concentration, light absorption rate is low and the penetration of light within the culture is ensured for all the microalgae. The higher the microalgae concentration, the more the microalgae cells absorb and reflect light, via a diffraction phenomenon, such that light penetrates less within the culture. Then, together with the increase of microalgae concentration, the light intensity emitted by the lighting device 6 increases to achieve the predefined value of light intensity at the light sensor 15.

The invention is not limited to the examples described and shown. In particular, in the illustrative embodiment described above of an apparatus and a method according to the invention, the apparatus 30 includes a unique light sensor 15, which is cost-effective but requires high homogeneity of the microalgae concentration. As a variant, an apparatus and a method according to the invention can involve several light sensors, which are then advantageously distributed peripherally on the wall of the vessel so as to detect the light intensity for several zones of the culture medium. In addition, in the example described above, reference has been made to Spirulina (*Arthrospira platensis*), but it is understood that the invention is applicable to the production of any type of microalgae, for example to the production of Chlorella or other specific aliment such as kombucha. The conditions of the culture are adapted to the specific type of microalgae which is cultured, in particular the temperature of the culture medium and the wavelengths and intensity of the light emitted by the lighting device.

**Claims**

1. Apparatus (30) for the production of microalgae, comprising a vessel (1) intended to receive a culture medium of the microalgae and a lighting device (6) intended to be positioned in the vessel inside the culture medium, the lighting device (6) being configured to emit light at least in a range of wavelengths useful for the photosynthesis of the microalgae, **characterized in that** the apparatus comprises a control system (15, 19) for automatically controlling the light intensity emitted by the lighting device (6) as a function of the concentration of microalgae in the vessel (1).

2. Apparatus according to claim **1, characterized in that** the control system comprises at least one light sensor (15) intended to receive a light intensity depending on the concentration of microalgae in the vessel (1) and a processing unit (19) configured to adjust the light intensity received by the light sensor (15) at a predefined value of light intensity by controlling the light intensity emitted by the lighting device (6).

3. Apparatus according to claim **2, characterized in that** the light sensor (15) is arranged to receive light emitted by the lighting device (6) and that has passed through the culture medium received in the vessel (1).

4. Apparatus according to either one of claims **2** or **3, characterized in that** the processing unit (19) is configured to determine the concentration of microalgae in the vessel (1) by comparison between the light intensity emitted by the lighting device (6) and the light intensity received by the light sensor (15).

5. Apparatus according to any one of the preceding claims, **characterized in that** the lighting device (6) is positioned centrally inside the vessel (1).

6. Apparatus according to any one of claims **2** to **5, characterized in that** the light sensor (15) is positioned on a peripheral wall (22) of the vessel (1), preferably on the outer face of the peripheral wall (22A) of the vessel (1).

7. Apparatus according to any one of the preceding claims, **characterized in that** the lighting device (6) comprises at least one LED (Light-Emitting Diode) emitting white light, preferably with a CCT (Color Correlated Temperature) of between about 3000K and 5000K.

8. Apparatus according to any one of the preceding claims, **characterized in that** it comprises a harvesting device (29) configured to ensure filtration of microalgae from a volume of the culture medium and allow the remaining culture medium to return in the vessel (1).

9. Apparatus according to claim **8, characterized in that** the harvesting device (29) comprises a membrane pump (9), a filtration membrane with a first pore size and a cup (16) with a second pore size less than the first pore size.

10. Apparatus according to any one of the preceding claims, **characterized in that** it comprises a heating device (10) intended to adjust the temperature of the culture medium received in the vessel (1).

11. Apparatus according to any one of the preceding claims, **characterized in that** it comprises an aeration and mixing device (28) intended to ensure homogeneity of temperature, homogeneity of microalgae distribution and gas exchange in the culture medium received in the vessel (1).

12. Method for the production of microalgae, comprising:

- filling a vessel (1) with a culture medium of the microalgae;
- activating a lighting device (6) positioned in the vessel inside the culture medium, the lighting device (6) being configured to emit light at least in a range of wavelengths useful for the photosynthesis of the microalgae;
- automatically controlling the light intensity emitted by the lighting device (6) as a function of the concentration of microalgae in the vessel (1).

13. Method according to claim **12, characterized in that** the automatic control of the light intensity emitted by the lighting device (6) as a function of the concentration of microalgae in the vessel (1) is performed by controlling the light intensity emitted by the lighting device (6) so as to adjust the light intensity received by at least one light sensor (15) at a predefined value of light intensity depending on the concentration of microalgae in the vessel (1).

14. Method according to claim **13, characterized in that** the light sensor (15) is arranged to receive light emitted by the lighting device (6) and that has passed through the culture medium received in the vessel (1).

15. Method according to any one of claims **12** to **14, characterized in that** the automatic control of the light intensity emitted by the lighting device (6) as a function of the concentration of microalgae in the vessel (1) is performed during a growing phase of the microalgae and/or during a harvesting phase of the microalgae.

**Fig.1**

**Fig.4**

Production days - step 1

**Fig.2**

**Fig.3**

Europäisches
Patentamt

European
Patent Office

Office européen
des brevets

# EUROPEAN SEARCH REPORT

Application Number

EP 18 18 2735

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/028977 A1 (NG K Y SIMON [US] ET AL) 4 February 2010 (2010-02-04) * paragraph [0024] - paragraph [0026] * * paragraph [0029] - paragraph [0030] * * paragraph [0035] - paragraph [0038] * * paragraph [0045] - paragraph [0049] * * figures 1-3 * | 1-15 | INV. C12M1/00 |
| A | US 2007/231886 A1 (KAHLERT WOLFGANG [DE] ET AL) 4 October 2007 (2007-10-04) * paragraphs [0011], [0014] * * paragraphs [0032], [0034], [0037], [0039], [0043] * * figures 1,2 * | 1-15 | |
| A | US 2015/210970 A1 (HELLINGWERF KLAAS JAN [NL] ET AL) 30 July 2015 (2015-07-30) * paragraph [0061] - paragraph [0064] * * paragraph [0079] * * figure 1 * | 1,6 | |
| A | KR 2008 0057849 A (KOREA RES INST OF BIOSCIENCE [KR]) 25 June 2008 (2008-06-25) * paragraph [0001] - paragraph [0022]; figures 2-4 * | 1 | TECHNICAL FIELDS SEARCHED (IPC) C12M |

The present search report has been drawn up for all claims

| Place of search | Date of completion of the search | Examiner |
|---|---|---|
| Berlin | 20 December 2018 | Cubas Alcaraz, Jose |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 18 2735

This annex lists the patent family members relating to the patent documents cited in the above-mentioned European search report.
The members are as contained in the European Patent Office EDP file on
The European Patent Office is in no way liable for these particulars which are merely given for the purpose of information.

20-12-2018

| Patent document cited in search report | | Publication date | Patent family member(s) | | Publication date |
|---|---|---|---|---|---|
| US 2010028977 | A1 | 04-02-2010 | NONE | | |
| US 2007231886 | A1 | 04-10-2007 | DE 102006014648 B3 | | 27-12-2007 |
| | | | US 2007231886 A1 | | 04-10-2007 |
| US 2015210970 | A1 | 30-07-2015 | EP 2899261 A1 | | 29-07-2015 |
| | | | NL 2012157 C | | 29-07-2015 |
| | | | US 2015210970 A1 | | 30-07-2015 |
| KR 20080057849 | A | 25-06-2008 | NONE | | |

EPO FORM P0459

For more details about this annex : see Official Journal of the European Patent Office, No. 12/82